# EUROPEAN PATENT APPLICATION

(11) **EP 4 458 960 A1**
(43) Date of publication of application: **06.11.2024**
(21) Application number: 23765754.9
(22) Date of filing: 17.02.2023
(51) Int. Cl.: C12N 9/06, C12N 15/70, C12N 1/21, C12P 13/02

(54) **ENGINEERING BACTERIUM FOR EXPRESSING ASPARTATE DEHYDROGENASE AND METHOD FOR PRODUCING VITAMIN B5 BY FERMENTATION**

(30) Priority: 07.03.2022 CN 202210214588
(71) Applicant: Institute Of Microbiology, Chinese Academy Of Sciences, Beijing 100101 (CN)
(72) Inventor: LIU, Shuwen, Beijing 100101 (CN); WEN, Tingyi, Beijing 100101 (CN); SUN, Jiahui, Beijing 100101 (CN); LI, Zhongcai, Beijing 100101 (CN); ZHANG, Yun, Beijing 100101 (CN); DENG, Aihua, Beijing 100101 (CN)
(74) Representative: Strych, Sebastian
(86) International application number: PCT/CN2023/076669
(87) International publication number: WO 2023/169176

(57) **Abstract**

The present application relates to the field of microorganisms, and specifically relates to Escherichia coli expressing aspartate dehydrogenase *aspDH* and a method for producing vitamin B5 by fermentation. Overexpression of the *aspDH* gene shows the best result by comparing the fermentation yield of VB5. Compared with the highly polluting chemical method for the production of vitamin B5, the biological method for the production of vitamin B5 of the present application has the advantages of renewable raw materials, easy treatment and resource utilization of waste residue, waste water and waste gas, and thus can be used in practice for the industrial production of vitamin B5, which is of significant application value.

## Description

### Technical Field

The present application relates to the field of microorganisms, and specifically relates to an engineering bacteria expressing aspartate dehydrogenase and a method for producing vitamin B5 by fermentation.

### Background

Vitamin B5 (VB5), also known as D-pantothenic acid, is a water-soluble vitamin, and is a component of coenzyme A and an acyl carrier protein. As an cofactor of 70 enzymes, the vitamin B5 participates in the metabolism of sugar, fat, protein, and energy, and plays an important role in regulating physiological metabolism. VB5 is mainly used for animal feed additives, food additives and pharmaceutical raw materials. With the discovery of new functions and the expansion of the application field of VB5, market demands of VB5 still tend to increase steadily.

China is the largest country in the production and export of VB5, the industrial production method of VB5 is a chemical synthesis. Enterprises basically use the Isobutyraldehyde-formaldehyde-hydrocyanic acid method to synthesize DL-pantolactone, the DL-pantolactone is further split by a chemical or enzymatic method to obtain L-pantolactone. Finally, theβ-alanine produced by using acrylonitrile as a raw material and the L-pantolactone synthesizes VB5. The main raw materials for chemical synthesis of VB5 are flammable, explosive, and highly toxic. Cyanic wastewater will be produced in the production process, and the wastewater is difficult to treat, leading to VB5 becoming a heavy pollution industry.

In recent years, China's economic development has entered into a new state of green environmental protection, and the impact of environmental protection on the VB5 industry has gradually emerged. Under large-scale and high-intensity environmental protection and management, the production of high-pollution VB5 enterprises have been limited and even ceased, and the market supply is short and the price is skyrocketed, which has restricted the health development of the downstream feed, food and pharmaceutical industries. Such a supply and demand situation will continue for a long time until there is a significant improvement in the highly polluting VB5 production technology, therefore, the innovation of VB5 green manufacturing technology for VB5 is urgent.

The microbial fermentation method for VB5 production not only use renewable glucose as a raw material, but also waste residues, waste water and waste gas formed in the production process are easy to be treated and utilized in a resourceful way, thereby effectively solving the problem of high pollution in the VB5 industry. The metabolic pathway of VB5 synthesis by microorganisms using glucose has a complex regulatory mechanism and the fermentation yield is extremely low.β-alanine is used as a C3 substrate to synthesize VB5 with D-Pantothenic acid. In order to increase the fermentation yield of VB5, it is necessary to exogenously add large amounts of β-alanine to the fermentation medium (Sahm, H. , et al. , (1999) Appl Environ Microb, 65, 1973-1979; Dusch, N. , et al. , (1999) Appl Environ Microb, 65, 1530-1539; Zhang, B. , et al. , (2019) Food Chemistry, 294, 267-275.) β-alanine can be produced by decarboxylation of L-Aspartic acid, therefore, enhancing the biosynthesis of aspartic acid is expected to increase the yield of VB5 produced by fermentation.

### Summary

In view of this, in the present application, three enzymes are respectively overexpressed in Escherichia coli for producing VB5 by means of fermentation, and it is found that AspDH is more beneficial to improve the fermentation yield of VB5 than AspC and AspA.

In order to achieve the purpose of the invention, the present application provides the following technical solutions:
In first aspect, the present application provides an application of enhanced expression of an aspartate dehydrogenase gene *aspDH* in the production of vitamin B5;
Preferably, the aspartate dehydrogenase gene *aspDH* is derived from *Delftia sp.* Cs1-4.

In some embodiments of the present application, the aspartate dehydrogenase gene *aspDH* has:
(I) a nucleotide sequence shown as SEQ ID No. 55; or
(II) a nucleotide sequence obtained by substituting, deleting or adding one or more bases to the nucleotide sequence shown as (I) and having the same or similar functions as the nucleotide sequence shown as (I); or
(III) a nucleotide sequence having at least 80% homology to the nucleotide sequence shown as (I) or (II).

In some embodiments of the present application further including:
(1) inserting a strong promoter and/or a strong RBS into a *cadA* gene, wherein the strong promoter is PgapA and the strong RBS is BCD2;
   preferably, the BCD2 has:
   (A) a nucleotide sequence shown as SEQ ID No. 2; or
   (B) a nucleotide sequence obtained by substituting, deleting or adding one or more bases to the nucleotide sequence shown as (A), and having the same or similar function as the nucleotide sequence shown as (A); or
   (C) a nucleotide sequence having at least 80% homology to the nucleotide sequence shown as (A) or (B);
      and/or
(2) expressing an *ilvGM* gene derived from E. coli BL21; and/or
(3) expressing a L-aspartate α-decarboxylase gene *panD* derived from *Bacillus licheniformis;* and/orpreferably, the L-aspartate α-decarboxylase gene *panD* derived from *Bacillus licheniformis* has:
   (A) a nucleotide sequence shown as SEQ ID No. 1; or
   (B) a nucleotide sequence obtained by substituting, deleting or adding one or more bases to the nucleotide sequence shown as (A), and having the same or similar function as the nucleotide sequence shown as (A); or
   (C) a nucleotide sequence having at least 80% homology to the nucleotide sequence shown as (A) or (B); and/or
(4) increasing copy number of *a panB* gene, *a panC* gene and/or *a panE* gene.
   In second aspect, the present application provides an expression vector, including an aspartate dehydrogenase *aspDH* gene;
   preferably, the aspartate dehydrogenase *aspDH* gene is derived from *Delftia sp.* Csl-4 ;
   preferably, the aspartate dehydrogenase *aspDH* gene has:
      (I) a nucleotide sequence shown as SEQ ID No. 56; or
      (II) a nucleotide sequence obtained by substituting, deleting or adding one or more bases to the nucleotide sequence shown as (I), and having the same or similar function as the nucleotide sequence shown as (I); or
      (III) a nucleotide sequence having at least 80% homology to the nucleotide sequence shown as (I) or (II).
   In some embodiments of the present application, the expression vector further including:
      (I) a strong promoter and/or a strong RBS;
         wherein the strong promoter is PgapA and the strong RBS is BCD2;
         preferably, the BCD2 has:
            (A) a nucleotide sequence shown as SEQ ID No. 2; or
            (B) a nucleotide sequence obtained by substituting, deleting or adding one or more bases to the nucleotide sequence shown as (A), and having the same or similar function as the nucleotide sequence shown as (A); or
            (C) a nucleotide sequence having at least 80% homology to the nucleotide sequence shown as (A) or (B);
               and/or
      (II) an *ilvGM* gene derived from E. coli BL21; and/or
      (III) a L-aspartate α-decarboxylase gene *panD* derived from *Bacillus licheniformis;* and/or
         preferably, the L-aspartate α-decarboxylase gene *panD* derived from *Bacillus licheniformis* has:
         (A) a nucleotide sequence shown as SEQ ID No. 1; or
         (B) a nucleotide sequence obtained by substituting, deleting or adding one or more bases to the nucleotide sequence shown as (A), and having the same or similar function as the nucleotide sequence shown as (A); or
         (C), a nucleotide sequence having at least 80% homology to the nucleotide sequence shown as (A) or (B); and/or
      (IV) a *panB* gene, a *panC* gene and/or a *panE* gene with increased copy number.
         In third aspect, the present application provides a host, wherein the host expresses an aspartate dehydrogenase gene *aspDH;*
         preferably, the aspartate dehydrogenase gene *aspDH* is derived from *Delftia sp.* Csl-4;
         preferably, the aspartate dehydrogenase gene *aspDH* has:
            (I) a nucleotide sequence shown as SEQ ID No. 56; or
            (II) a nucleotide sequence obtained by substituting, deleting or adding one or more bases to the nucleotide sequence shown as (I), and having the same or similar function as the nucleotide sequence shown as (I); or
            (III) a nucleotide sequence having at least 80% homology to the nucleotide sequence shown as (I) or (II).
   In some embodiments of the present application, the host further including:
      (I) a strong promoter and/or a strong RBS;
         wherein the strong promoter is PgapA and the strong RBS is BCD2;
         preferably, the BCD2 has:
            (A) a nucleotide sequence shown as SEQ ID No. 2; or
            (B) a nucleotide sequence obtained by substituting, deleting or adding one or more bases to the nucleotide sequence shown as (A), and having the same or similar function as the nucleotide sequence shown as (A); or
            (C) a nucleotide sequence having at least 80% homology to the nucleotide sequence shown as (A) or (B);
               and/or
      (II) an *ilvGM* gene derived from E. coli BL21; and/or
      (III) a L-aspartate α-decarboxylase gene *panD* derived from *Bacillus licheniformis;* and/or
         preferably, the L-aspartate α-decarboxylase gene *panD* derived from *Bacillus licheniformis* has:
         (A) a nucleotide sequence shown as SEQ ID No. 1; or
         (B) a nucleotide sequence shown as (A) obtained by substitution, deletion or addition of one or more bases, and having the same or similar function as the nucleotide sequence shown as (A); or
         (C) a nucleotide sequence having at least 80% homology to the nucleotide sequence shown in (A) or (B); and/or
      (IV) a *panB* gene, a *panC* gene and/or a *panE* gene with increased copy number.

In some embodiments of the present application, the host is transfected or transformed the expression vector according to claim 4 or 5.

Preferably, the host is derived from E. coli, more preferably, the host is derived from E. coli K12, and more preferably, the host is derived from E. coli K12 MG1655 strain.

In fourth aspect, the present application provides the application of the expression vector or the host in the production of vitamin B5.

In fifth aspect, the present application provides a method for the production of vitamin B5, wherein the host is used as a fermentation strain, fermented, the fermentation broth is collected, and the supernatant is centrifuged to obtain vitamin B5.

The present application discloses an Escherichia coli (E. coli) expressing an aspartate dehydrogenase gene *aspDH,* and a method for producing vitamin B5 (VB5) by fermentation. The present application respectively enhances three L-Aspartic acid synthesis routes in VB5 engineering bacteria. The three L-Aspartic acid synthesis routes are respectively that aspartate aminotransferase encoded by an *aspC* gene transfers an amino group of glutamic acid to oxaloacetate to produce L-Aspartic acid and ketoglutaric acid; an aspartate ammonialyase encoded by an *aspA* gene catalyzes the production of aspartic acid from ammonium and fumaric acid; and aspartate dehydrogenase encoded by an *aspDH* gene catalyzes the synthesis of aspartic acid from oxaloacetate and ammonium. By comparing the fermentation yield of VB5, overexpression of the *aspDH* gene shows the best effect. Compared with the high pollution chemical method for the production of vitamin B5, the biological method of the present application for producing vitamin B5 has the advantages of renewable raw materials, easy treatment and resource utilization of waste residues, waste water and waste gas, etc. Therefore, it can be used for the industrial production of vitamin B5 in practice, and has an important application value.

### Detailed Description of Embodiments

The present application discloses an engineering bacterium expressing aspartate dehydrogenase and a method for producing Vitamin B5 by fermentation, and the skilled in the art can refer to the contents of this paper and improve the process parameters appropriately. It should be noted that all similar substitutions and modifications are obvious to the skilled in the art, and they are all considered to be included in the present application. The method and application of the present application have been described through preferred embodiments, and relevant personnel can obviously modify or appropriately change and combine the method and application described herein without deviating from the content, spirit and scope of the present application to achieve and apply the technology of the present application.

There are two pathways for producing Aspartic acid in E. coli, one of pathway is the aspartate aminotransferase encoded by *aspC* gene transfering the amino group of glutamate to oxaloacetic acid to produce L-aspArtic acid and ketoglutaric acid; The other is the aspartate ammonia lyase encoded by the *aspA* gene catalyzing the production of Aspartic acid from ammonium and fumaric acid. In addition, an aspartate dehydrogenase (AspDH) that catalyzes the synthesis of Aspartic acid from oxaloacetate and ammonium has been found in some archaea. In the present application, the above three enzymes are respectively overexpressed in Escherichia coli for producing VB5 through fermentation, and it is found that AspDH is more advantageous than AspC and AspA for improving the fermentation yield of VB5.

In order to break through the metabolic bottleneck of efficient synthesis of VB5, the inventors compared three pathways to improve the synthesis of aspartic acid, and found that the heterologous aspartic acid dehydrogenation (encoded by *aspDH* gene) was more suitable for its own aspartic acid transamination pathway (encoded by *aspC* gene) and aspartic acid ammonia cleavage pathway (encoded by *aspA* gene).

An aspartate dehydrogenase (AspDH) that catalyzes the reversible reaction of oxaloacetate with ammonium root and NAD(P)H to form aspartic acid with water and NAD(P)⁺, and is produced by one or more of the following microorganisms: *Pseudomonas aeruginosa, Klebsiella pneumoniae, Serratia proteamaculans, Thermotoga maritima, Chromohalobacter salexigens, Acinetobacter baumannii, , Delftia sp.* Csl-4 , *Ochrobactrum anthropi, Caulobacter sp.* , *Methanohalophilus mahii, Dinoroseobacter shibae, Methanosphaerula palustris, and Methanobrevibacter ruminantium,* etc.

In terms of expressed aspartate dehydrogenase, the present application uses a strong promoter to regulate the expression of the *aspDH* gene. The promoter may be a strong promoter may be the following promoters or mutants thereof: L promoter, trc promoter, T5 promoter, lac promoter, tac promoter, T7 promoter, or *gap A* promoter. In addition, the present application uses a more active RBS sequence to regulate translation initiation of the *aspDH* gene.

The present application integrates the above high intensity translation initiation and transcription initiation regulated *aspDH* gene into the E. coli chromosome for VB5 production by fermentation method to realize the expression of the exogenous gene. The integration site is the *cadA* gene, which encodes lysine decarboxylase and theoretically has no effect on VB5 biosynthesis.

The effects of VB5 synthesis of overexpressing *aspA, aspC* and *aspDH* genes were compared by integrating *aspA* and *aspC* genes, respectively, at the same *cadA* gene locus on the chromosome of E. coli where VB5 was produced by the fermentation method, and by using the same promoter to regulate transcription.

The E. coli described in the present application for VB5 production by fermentation also overexpresses *panB, panC* and *panE* genes on the VB5 terminal synthesis pathway. The *panB* gene of E. coli encodes a ketopantothenate hydroxymethyltransferase, which catalyzes the addition of a methyl group to the substrate a-ketoisovaleric acid to form Ketopantoic acid. Ketopantoic acid is reduced to pantoic acid by ketopantothenate reductase encoded by the *panE* gene. The pantothenate synthase encoded by *the panC* gene further catalyzes the condensation of pantothenate and β-alanine to form VB5.

The Escherichia. coli used in the present application is strain K12 MG1655, which has a mutated inactivation of the *ilvG* gene. Therefore, the present application introduces the active *ilvG* gene of E. coli BL21, which improves the supply of precursor acetolactate synthesis for VB5. In the present application, the *ilvG*⁺*M* gene derived from Escherichia coli BL21 is inserted into the chromosome of E. coli K12 MG1655, a strong trc promoter is used to regulate the initiation of transcription of *ilvG*⁺*M*, and a terminator Ter is used to regulate the termination of transcription of *ilvG*⁺*M.* The insertion site of the *ilvG*⁺*M* gene on the chromosome is the coding sequence of the *avtA* gene, which leads to the inactivation of AvtA and weakens the synthesis of valine, thereby weakening the competitive pathway of VB5 and facilitating VB5 biosynthesis.

The *panD* gene derived from *Bacillus licheniformis* was also integrated on the *avtA* gene of the engineered bacteria. The same strong promoters PPL and BCD2 were used to regulate transcription and translation initiation, respectively.

A method for producing VB5 by fermentation, the culture medium contains a carbon source, a nitrogen source, inorganic ions, antibiotics and other nutritional factors. As the carbon source, sugars such as glucose, lactose, galactose, etc can be used. As an inorganic nitrogen source, inorganic nitrogen source such as ammonia water, ammonium sulfate, ammonium phosphate, ammonium chloride, etc can be used; and as an organic nitrogen source, organic nitrogen source such as corn syrup, soybean meal hydrolysate, hair powder, yeast extract, peptone, etc can be used. The inorganic ions include one or more of iron, calcium, magnesium, manganese, molybdenum, cobalt, copper, potassium, and other ions.

The experimental methods in the following embodiments are conventional methods unless otherwise specified. The experimental materials used in the following embodiments were purchased from a conventional biochemical reagent store, unless otherwise specified. The quantitative tests in the following embodiments were set up for three repetitions, and the results were averaged. Unless otherwise specified, the technical means used in the following embodiments are conventional means well known to the skilled in the art and commercially available instruments and reagents, Please refer to the Experimental Guide to <Molecular Cloning (3rd Edition) > (Science Press), <Microbiology Experiment (4th Edition)> (Higher Education Press) as well as the manufacturer's manuals of the corresponding instruments and reagents, etc.

If the sequence in the specification is inconsistent with the sequence in the sequence list, the sequence in the specification will prevail.

Escherichia. coli K12 MG1655: ATCC number 700926. pACYC184 plasmid: NEB, cat. NO. E4152S. plasmid pcas9 was purchased from Addgene, cat. NO 62225; plasmid pTargetF was purchased from Addgene, cat. NO 62226.

The present application is further illustrated below in conjunction with the embodiments:
Embodiment 1 Detection methods

The yield of the fermentation broth VB5 was quantitatively determined using HPLC and the specific method is as follows: The supernatant of the fermentation broth was diluted with purified water to the appropriate concentration and filtered through 0.22µm membrane. The chromatographic column was Agilent ZORBAX SB-Aq, 4.6×250 mm with a column temperature of 30 °C, the detection wavelength was 210 nm, the flow rate of mobile phase was 1 mL/min, the mobile phase was 3.12 g/L NaH₂PO₄-2H₂O, and the pH was adjusted to 2.2 with phosphoric acid, and standard curves for concentration and optical absorbance of 0.1-0.5g/L calcium pantothenate were determined using calcium pantothenate (VB5) purchased from sigma as standard.

### Embodiment 2 Construction of Engineered Bacteria for Fermentation Production of VB5

The nucleotide sequence amplified by PCR was shown in SEQ ID No. 3 using high-fidelity polymerase KAPA HiFiTM HotStar with P1 and P2 as primers and genomic DNA of wild-type E. coli strain K12 MG1655 as template, in which 10nt-45nt was the promoter trc, 74nt-868nt was the coding sequence of *panB* gene, and 880nt-1731nt is the coding sequence *of panC* gene. Primer P1 was designed to introduce the strong promoter trc, and primers P1 and P2 were designed to introduce BamHI and SphI restriction endonuclease sites at the 5' end of primer P1 and P2, respectively. The PCR program was: denaturation at 98°C for 30 seconds, annealing at 65°C for 15 seconds, extension at 72°C for 90 seconds, and 26 cycles, and the *P_{trc}*-*panB*C gene fragment of about 1800 bp was obtained. 
P1 : 5'- CGCGGATCC
CAATTAATCATCCGGCTCGTATAATGTGTGGAGCACAACATCAATTTATCAGGA
 (As shown in SEQ ID NO: 10, the underlined sequence is the BamHI cleavage recognition site, and the italic is the sequence of the promoter trc)
P2 : 5'- ACATGCATGC CCTGTGTTAT GACAGATGAC -3'
(As shown in SEQ ID NO: 11, the underlined sequence is the SphI restriction recognition site)

The *P_{trc}-panB*C product amplified by PCR was identified and recovered by gel electrophoresis, and then was double digested using BamHI and SphI. The pACYC184 plasmid were simultaneously double digested with restriction endonucleases BamHI and SphI. The double-cleaved *P_{trc}-panB*C and pACYC184 plasmids were recovered by gel electrophoresis, ligated with T4 ligase, and the ligation products were chemically transformed into E. coli DH5α competent cells, which were resuscitated for 1 hour and coated on the chloramphenicol plates. The coated plates were placed in a 37°C incubator for 12 hours, single colonies were picked for passaging, and the recombinant plasmids were extracted and sequenced to obtain the correct recombinant plasmid pACYC184-panBC.

The genome of E.coli K12 MG1655 was used as a template, and the sequence obtained by PCR amplification using P3 and P4 as primers was shown in SEQ ID No.4, in which 11nt-45nt is the promoter of PJ23119, 66nt-977nt is the coding sequence *of panE* gene, and 988nt-1731nt is the terminator sequence. The promoter PJ23119 was designed on the amplification primer P3, the sequence of terminator L3S2P56 was designed on the primer P4, and SphI and BsaBI restriction endonuclease sites were designed on the 5' end of primers P3 and P4, respectively. The PJ23119-panE product obtained by amplification was identified using the PCR reaction conditions described above and recovered by gel electrophoresis, and then digested with SphI and BsaBId, and at the same time the pACYC184-Ptrc-*panB*C plasmid was double-digested with SphI and BsaBI. The plasmid PJ23119-panE and pACYC184-panBC after enzyme digestion were recovered by gel electrophoresis, and after ligation using T4 ligase, the ligation products were chemically transformed into E.coli DH5α competent cells, which were resuscitated for 1 hour and were coated on the chloramphenicol plates. The coated plate was placed in a 37°C incubator for 12 hours, and single colonies were picked for passaging, and the recombinant plasmids were extracted and sequenced to obtain the correct recombinant plasmid, pACYC184-panBCE, thus obtaining a recombinant plasmid for overexpressing a vitamin B5 terminal synthesis pathway gene. 
P3 : 5'- ACATGCATGC
ttgacagctagctcagtcctaggtataatgctagcGTTGCGGGTGAGGAGGAACA
 (As shown in SEQ ID NO: 12, the underlined sequence is the SphI recognition site, and the italicized sequence is the sequence of promoter J23119) 
P4 : 5’- CTCGATTTAGATCCCAAAACGAA AAAAGACGCGCTTTTCAGC
GTCTTTTTTC GAAAATTAGT CTCTTCACTA CCAGGGATGA CTATCGAG
 (As shown in SEQ ID NO: 13, the underlined sequence is a BsaBI restriction recognition site, and the italic is the L3S2P56 terminator sequence)

Application of a reported CRISPR-Cas9 gene editing system including pCas9 and pTargetF vectors (Jiang, Y., Chen, B., Duan, C. L., Sun, B. B., Yang, J. J., and Yang, S. (2015) Multigene Editing in the Escherichia coli Genome via the CRISPR-Cas9 System, Appl Environ Microb 81, 2506-2514.) .

The pTargetF vector was mutated using the Q5^{®} Site-Directed Mutagenesis Kit (Item No. E0552S) from NEB, with primers P5 and P6 designed according to the kit instructions. The mutated N20 sequence is CTTTTCCAAGC TGGGTCTACC, targeting the *avtA* gene. The mutated pTargetF was named pTargetFavtA.
P5: TGGGTCTACCG TTTTAGAGCT AGAAATAGC (as shown in SEQ ID NO.14);
P6: GCTTGGAAAG GACTAGTATT ATACCTAGG (as shown in SEQ ID NO.15);
P7:CG GACTGGAAGA AGATCTG (as shown in SEQ ID NO.16);
P8:TTTCTTAGAC GTCGGAATTGAGACTCATGCACAGCACGA (as shown in SEQ ID NO.17);
P9:TCGTGCTGTGCATGAGTCTCAATTCCGACGTCTAAGAAAC (as shown in SEQ ID NO.18);
P10:GATCTCCTTT TTAAGTGAAC TTGGGGTCAG TGCGTCCTGC TGAT (as shown in SEQ ID NO. 19);
P11:ATCAGCAGGACGCACTGACCCCAAGTTCACTTAAAAAGGAGAT C (as shown in SEQ ID NO.20);
P12:TGCCGTTCATATTGGTGATGCAAAAAACCCCTCAAGACC (as shown in SEQ ID NO.21);
P13: GGTCTTGAGGGGTTTTTTGC ATC ACC AAT ATGAACGGC A (as shown in SEQ ID NO.22);
P14:GCTGATAGAG CTGCTTGGT (as shown in SEQ ID NO.23);
P15: GGAGCTACTC ACACTGCTTG (as shown in SEQ ID NO.24);
P16: CGCATACATT GATGCGTATG (as shown in SEQ ID NO.25).

The licheniformis aspartate α-decarboxylase gene *panD* (as shown in SEQ ID No. 1) derived from *Bacillus licheniformis* was synthesized at Gene Synthesis company, and the XbaI and HindIII restriction endonuclease sequences were removed by synonymous codon substitutions during the above *panD* gene sequences was customized and synthesized. When the above *panD* gene sequences was customized and synthesized, the same BCD2 sequences (as shown in SEQ ID No. 2) were simultaneously synthesized before each *panD* sequence, meanwhile XbaI and HindIII restriction enzyme cleavage sites were added at both ends of the BCD2-panD sequences. The synthesized sequence was ligated into the vector. The above synthesized BCD2-panD vector and pET28a(+) plasmid were double digested with restriction endonucleases XbaI and HindIII, and the gene fragment of BCD2-panD after enzyme digestion and linearized vector segment were recovered by gel electrophoresis, and the two fragments were further ligated using T4 ligase, and the ligation products were transformed into E. coli DH5α competent cells, and LB plates containing 50 mg/L kanamycin were used to screen the transformants containing the recombinant plasmid. The plasmids were extracted after transformant amplification and sent for sequencing to verify that the correct plasmid, pET28a-BCD2-*panDBl*, was obtained.

The upstream sequence of the *avtA* gene was amplified using primers P7 and P8, the PL promoter was amplified using primers P9 and P10, the BCD2-*panDBl*-Ter gene fragment was obtained by amplification using primers P11 and P12 with pET28a-BCD2-*panDBl* as a template, and the downstream sequence of the *avtA* gene was amplified using primers P13 and P14. The above four fragments were ligated by overlapping PCR to obtain DonorBl, assemblage of four DNA fragments (as shown in SEQ ID No. 5), which was used as the template for gene editing. Among them, 1nt-312nt of SEQ ID No.5 is the upstream sequence of target gene *avtA* gene, 313nt-474nt is the PL promoter, 475nt-560nt is the BCD2 sequence, 560nt-943nt is the panDBl sequence, 944nt-995nt is the terminator sequence, and 996-1261nt is the downstream sequence of *avtA* gene.

The pCas9 plasmid was transformed into MG1655 and coated on the kanamycin-resistant plates containing 50 mg/L kanamycin, and cultured at 30°C to obtain strain MG655/pCas9. MG1655/pCas9 bacterial moss was picked in 50 mL of kanamycin-containing LB in 500 mL shake flasks, cultured at 30°C at 220 rpm. When the culture medium had OD₆₀₀ of 0.2, arabinose with a final concentration of 10 mM was added for induction, and when the culture medium had OD₆₀₀ of 0.45, competent cells were prepared. Two microliters of pTargetFavtA plasmid and 10 microliters of DonorBs template DNA were taken and electro-transformed into MG655/pCas9 competent cells, which were coated on the double-resistant plate containing 50 mg/L kanamycin and 50 mg/L spectacularinomycin and incubated at 30°C. Single colonies integrating PPL-BCD2 panD-Ter on the *avtA* gene were identified using primers P15 and P16, and the correctly sized PCR product was verified by sequencing. The single colonies with correct sequencing were selected and cultured by adding 0.2 mM IPTG to eliminate the pTargetFavtA plasmid, respectively, to obtain the engineered bacterium E. coli MG1655 *avtA*:*panDBl*/pCas, and still prepare competent cells and ready for use according to the above method.

Engineering bacteria E. coli MG1655 avtA:panDBl/pCas was inoculated to non-resistant LB liquid medium, cultured at 37 °C for 12 hours, then the medium was diluted and coated on LB plate. The engineering bacteria *E. coli* MG1655 *avta: panDBL* with pCas plasmid eliminated were obtained respectively. The gene *panD* is inserted into the coding sequence of the chromosome *avtA* gene, which leads to the inactivation of AvtA and weakens the valine competitive metabolic pathway.

Wild-type Escherichia coli K12 MG1655 has a mutation in the *ilvG* gene, which encodes an inactive acetolactate synthase. In the present application, the active *ilvG* gene of E. coli BL21 was introduced on the chromosome of E. coli MG1655, which improved the synthesis of the precursor acetolactate of VB5. The *ilvG*⁺*M* gene derived from E. coli BL21 was inserted into the chromosome of E. coli K12 MG1655 and the trc strong promoter was used to regulate the transcription initiation of *ilvG*⁺*M* and the terminator Ter was used to regulate the transcription termination of *ilvG*⁺*M.* The *ilvG*⁺*M* gene was integrated into another N20 target sequence of the *avtA* gene. The pTargetF vector was mutated using the Q5^{®} Mutagenesis Kit and primers P17 and P18. pTargetF was named pTargetFavtA1 after the mutation.
P17: ACGGTCCACAG TTTTAGAGCT AGAAATAGC (as shown in SEQ ID NO.26);
P18: CGTAGTTACA GACTAGTATT ATACCTAGG (as shown in SEQ ID NO.27);
P19: GGCAGAAAAT CAGCCAGTTC (as shown in SEQ ID NO.28);
P20:TCCACACATTATACGAGCCG GATGATTAAT TGTCAAGAAC TCTGTAGCAA GGAAGG (as shown in SEQ ID NO.29);
P21:TTGA CAATTAATCATCCGGCTCGTATAATGTGTGGA CAAGATT CAGGACGGGG AAC (as shown in SEQ ID NO.30);
P22:CGAAAAAAGACGCTCTAAAA GCGTCTCTTT TCTGGTATAT TCCTTTTGCG CTCAG (as shown in SEQ ID NO.31);
P23: CAGAAAAGAGACGCTTTTAGAGCGTCTTTTTTCGTTTTGGAGCT ACTC ACACTGCTTG (as shown in SEQ ID NO.32);
P24:GCCAATATGC AGATGCTCATGAGCATCTGCATATTGGC (as shown in SEQ ID NO.33);
P25:CACGTTCGGA TATGAACTG (as shown in SEQ ID NO.34);
P26:CGTCAAGCTT CAGCAACTC (as shown in SEQ ID NO.35).

The upstream sequence of the *avtA* gene was amplified using primers P19 and P20, the *ilvG+M* sequence of E. coli BL21 was amplified using primers P21 and P22, and the downstream sequence of the *avtA* gene was amplified using primers P23 and P24. The trc promoter TTGACAATTAATCATCCGGCTCGTATAATGTGTGTGGA was introduced by primers P20 and P21. The terminator sequence CCAGAAAAGAGAGACGCTTTTAGAGAGCGTCTTTTTTTTCGTTTT was introduced by primers P22 and P23. The above three fragments were ligated using overlapping PCR to obtain the assembled DonorilvGM (as shown in SEQ ID No.6), which used as a template for gene editing. 1-305nt of SEQ ID No.6 is the upstream sequence of the target gene *avtA,* 306nt-341nt is the trc promoter, 367nt-2013nt is the coding sequence of the *ilvG*⁺ gene derived from E.coli BL21, 2010nt-2273nt is the coding sequence of *ilvM* gene, 2274-2328 is the terminator sequences, and 2329-2629 is the downstream sequences of the *avtA* gene.

Two microliters of pTargetFavtA1 plasmid and 10 microliters of DonorilvGM template DNA were taken and electro-transformed into *E. coli* MG1655 *avtA*:*panDBl*/pCas competent cells, which coated on the double-resistant plates containing 50 mg/L kanamycin and 50 mg/L spectacularinomycin, and incubated at 30°C. Single colonies integrating Ptrc-ilvG⁺M-Ter on the *avtA* gene were identified using primers P25 and P26, and the PCR product with correct size was verified by sequencing. Single colonies with correct sequencing were selected and cultured with 0.2 mM IPTG to eliminate the pTargetFavtA1 plasmid. Non-resistant LB liquid medium was further added to, cultured at 37°C for 12 h, then diluted and coated on LB plates, respectively, to obtain the engineered bacteria E. coli *MG1655 avtA:panDBl-ilvG*⁺*M* that eliminated the pCas plasmid. The synthesis of the VB5 precursor acetolactate was improved by the integration of active *ilvG*⁺*M* on the chromosome.

The N20 sequence of the pTargetF vector was mutated using the Q5^{®} Mutagenesis Kit and primers P27 and P28 above. pTargetF was named pTargetFcadA after the mutation.
P27: TCATATCTCCG TTTTAGAGCT AGAAATAGC (as shown in SEQ ID NO.36);
P28: CTATGAACGT GACTAGTATT ATACCTAGG (as shown in SEQ ID NO.37);
P29: GTTGCGT GTTCTGCTTC ATC (as shown in SEQ ID NO.38);
P30: CCAGTTGGTG TTAATGTTTT GCTCCCAACA CATGGGACA (as shown in SEQ ID NO.39);
P31: TGTCC CATGTGTTGG GAGCA AAACATTAACACCAACTGG (as shown in SEQ ID NO.40);
P32: CTCCTTAGCA TGATTAAGAT GGTGAATAAA AGGTTGCCTGT (as shown in SEQ ID NO.41);
P33: ACAGGCAACCTT TTATTCACCATCTTAATCATGCTAAGGAG (as shown in SEQ ID NO.42);
P34: GCTAATTTCT TCGCACAGCT GGACCAAAAC GAAAAAAGAC G (as shown in SEQ ID NO.43);
P35: CGTCTTTTTTCGTTTTGGTCCAGCTGTG CGAAGAAATT AGC (as shown in SEQ ID NO.44);
P36: TCGTCAGTGG TCTGCTTGA (as shown in SEQ ID NO.45);
P37: CTAC TCTTGCGTTG ACCTGA (as shown in SEQ ID NO.46);
P38:GTGACCAGGA GTACAGAAAG (as shown in SEQ ID NO.47).

The E. coli MG1655 genome was used as a template to amplify the upstream sequence of the *cadA* gene using primers P29 and P30, primers P31 and P32 were used to amplify gapA promoter and primers P35 and P36 were used to amplify the downstream sequence of *cadA* gene. The *aspDH* gene containing RBS and terminator was synthesized from Gene Synthesis company, and the RBS-aspDH-Ter sequence was amplified by using primers P33 and P34. The above four fragments were ligated by overlapping PCR to obtain the assembled DonoraspDH (as shown in SEQ ID No. 7), which was used as a template for gene editing. 1-210nt of SEQ ID No. 7 is the upstream sequence of the target gene, *cadA* gene, 211nt-480nt is the gapA promoter, and 481nt-509nt is the RBS sequence. 510nt-1307nt is the coding sequence of the *aspDH* gene derived from Csl-4 of C. dell'arte (as shown in SEQ ID No. 56), 1308nt-1360nt is the terminator sequence, and 1361-1535 is the downstream sequence of the *cadA* gene.

Two microliters of pTargetFcadA plasmid and 10 microliters of DonoraspDH template DNA were taken and electro-transformed into *E. coli* MG1655 *avtA:panDBl-ilvG*⁺*M*/pCas competent cells, which coated on double-resistant plates containing 50 mg/L kanamycin and 50 mg/L spectacularinomycin, and incubated at 30°C. Single colonies integrating PgapA-aspDH-Ter on the *cadA* gene were identified by using primers P37 and P38, and the PCR product with correct size was verified by sequencing. Single colonies with correct sequencing were selected and cultured with 0.2 mM IPTG to eliminate the pTargetFcadA plasmid. Non-resistant LB liquid medium was further added to, cultured at 37°C for 12 h, then diluted and coated on LB plates to obtain the engineered bacteria *E. coli* MG1655 *avtA:panDBl-ilvG*⁺*M-aspDH* that eliminated pCas plasmid.

The E. coli MG1655 genome was used as a template to amplify the upstream sequence of the *cadA* gene using primers P29 and P30, primers P31 and P39 were used to amplify *gapA* promoter, primers P40 and P41 were used to amplify *aspC* gene, and primers P42 and P36 were used to amplify the downstream sequence of *cadA* gene. The above four fragments were ligated by overlapping PCR to obtain the assembled DonoraspC (as shown in SEQ ID No. 8), which was used as a template for gene editing. 1-210nt of SEQ ID No. 8 is the upstream sequence of the target gene *cadA* gene, 211nt-480nt is the *gapA* promoter, 611nt-1801nt is the coding sequence of *aspC* gene, and 1992-2166 are downstream sequences of the *cadA* gene.

Two microliters of pTargetFcadA plasmid and 10 microliters of DonoraspC template DNA were taken and electro-transformed into *E. coli* MG1655 *avtA*:*panD*B*l-ilvG*⁺*M*/pCas competent cells, which coated on double-resistant plates containing 50 mg/L kanamycin and 50 mg/L spectacularinomycin, and incubated at 30°C. Single colonies integrating PgapA-aspC on the *cadA* gene were identified by using primers P37 and P38, and the PCR product with correct size was verified by sequencing. Single colonies with correct sequencing were selected and cultured with 0.2 mM IPTG to eliminate the pTargetFcadA plasmid. Non-resistant LB liquid medium was further added to, cultured at 37°C for 12 h, then diluted and coated on LB plates to obtain the engineered bacteria *E. coli* MG1655 *avtA*:*panDBl-ilvG*⁺*MaspC* that eliminated pCas plasmid.
P39: GAGATTGCTC TGGAAGGTAT AGTGAATAAA AGGTTGCCTG T (as shown in SEQ ID NO.48);
P40: ACAGGCAACCTT TTATTCACTATACCTTCC AGAGCAATCT C (as shown in SEQ ID NO.49);
P41: GCTAATTTCT TCGCACAGCT CCTGGATTTC TGGCAAAGTG (as shown in SEQ ID NO.50);
P42: CACTTTGCC AGAAATCCAG GAGCTGTG CGAAGAAATT AGC (as shown in SEQ ID NO.51).

The E. coli MG1655 genome was used as a template to amplify the upstream sequence of the *cadA* gene using primers P29 and P30, primers P31 and P43 were used to amplify *gapA* promoter, primers P44 and P45 were used to amplify *aspA* gene, and primers P46 and P36 were used to amplify the downstream sequence of *cadA* gene. The above four fragments were ligated by overlapping PCR to obtain the assembled DonoraspA (as shown in SEQ ID No. 9), which was used as a template for gene editing. 1-210nt of SEQ ID No. 9 is the upstream sequence of the target gene *cadA* gene, 211nt-480nt is the *gapA* promoter, 504nt-1940nt is the coding sequence of *aspA* gene, and 2004-2178 are downstream sequences of the *cadA* gene.

Two microliters of pTargetFcadA plasmid and 10 microliters of DonoraspA template DNA were taken and electro-transformed into *E. coli* MG1655 *avtA*:*panDBl-ilvG*⁺*M*/pCas competent cells, which coated on double-resistant plates containing 50 mg/L kanamycin and 50 mg/L spectacularinomycin, and incubated at 30°C. Single colonies integrating PgapA-aspA on the *cadA* gene were identified by using primers P37 and P38, and the PCR product with correct size was verified by sequencing. Single colonies with correct sequencing were selected and incubated with 0.2 mM IPTG to eliminate the pTargetFcadA plasmid. Non-resistant LB liquid medium was further added to, cultured at 37°C for 12 h, then diluted and coated on LB plates to obtain the engineered bacteria E. coli MG1655 *avtA*:*panDBl-ilvG*⁺*M-aspA* that eliminated pCas plasmid.
P43: GAACCTTCTT TTTCAAGCTG CGTGAATAAA AGGTTGCCTG T (as shown in SEQ ID NO.52);
P44: ACAGGCAACCTT TTATTCACGCAGCTTGAAAAA GAAGGTTC (as shown in SEQ ID NO.53);
P45: GCTAATTTCT TCGCACAGCT CTGCTCACAA GAAAAAAGGC (as shown in SEQ ID NO.54);
P46: GCCTTTTTTC TTGTGAGCAGAGCTGTG CGAAGAAATT AGC (as shown in SEQ ID NO.55).

The above constructed vector pACYC184-panBCE was transformed into the above engineered bacteria *E. coli* MG1655 *avtA:panDBl-ilvG*⁺*M-aspDH*, *E. coli* MG1655 *avtA:panDBl-ilvG*⁺*M-aspC and E. coli* MG1655 *avtA:panDBl-ilvG*⁺*M-aspA*, respectively. *E. coli* MG1655 *avtA:panDBl-ilvG*⁺*M-aspDH*/*pACYC184-panBCE*, *E. coli* MG1655 *avtA:panDBl-ilvG*⁺*M-aspC*/*pACYC184-panBCE* and *E. coli* MG1655 *avtA:panDBl-ilvG*⁺*M-aspA*/*pACYC184-panBCE* were obtained respectively for fermentation to produce VB5.
**SEQ ID No.1** 
ATGTACCGTA CGTTAATGAG CGCAAAACTT CACAGAGCGA
GAGTGACGGA AGCCAATTTG
AACTACGTCG GCAGCGTGAC AATTGATGAA GATTTGCTGG ATGCTGTCGG
AATGATGGCA
 
AATGAAAAAG TGCAAATTGT GAATAATAAT AACGGGGCCC
GGCTGGAAAC GTACATTATT
CCCGGTGAAA GGGGCAGCGG CGTCGTTTGT TTAAACGGAG
CTGCCGCCCG CCTTGTCCAG
GTTGGAGATG TCGTCATCAT CGTGTCTTAT GCGATGATGT CTGAAGAGGA
AGCAAAGACC
CATAAGCCGA AGGTTGCCGT TTTGAACGAG AGAAACGAAA
TCGAGGAAAT GCTGGGTCAG
GAGCCAGCCC GTACCATTCT GTAA
**SEQ ID No.2** 
CCAAGTTCACTTAAAAAGGAGATCAACAATGAAAGCAATTTTCGTACTGAA
ACATCTTAATCATGCTAAGGAGGTTTTCTAATG
**SEQ ID No.3** 
CGCGGATCC TTGA CAATTAATCATCCGGCTCGTATAATGTGTGGA
GCACAACA
TCAATTTATCAGGATACGTTATGAAACCGACCACCATCTCCTTACTGCAGAA
GTACAAAC
AGGAAAAAAAACGTTTCGCGACCATCACCGCTTATGACTATAGCTTCGCCA
AACTCTTTG
CTGATGAAGGGCTTAACGTCATGCTGGTGGGCGATTCGCTGGGCATGACGG
TTCAGGGGC
ACGACTCCACCCTGCCAGTTACCGTTGCCGATATCGCCTACCACACTGCCG
CCGTACGTC
GCGGCGCACCAAACTGCCTGCTGCTGGCTGACCTGCCGTTTATGGCGTATG
CCACGCCGG
AACAAGCCTTCGAAAACGCCGCAACGGTTATGCGTGCCGGTGCTAACATG
GTCAAAATTG
AAGGCGGTGAGTGGCTGGTAGAAACCGTACAAATGCTGACCGAACGTGCC
GTTCCTGTAT
GTGGTCACTTAGGTTTAACACCACAGTCAGTGAATATTTTCGGTGGCTACA
 
AAGTTCAGG
GGCGCGGCGATGAAGCGGGCGATCAACTGCTCAGCGATGCATTAGCCTTAG
AAGCTGCTG
GGGCACAGCTGCTGGTGCTGGAATGCGTGCCGGTTGAACTGGCAAAACGT
ATTACCGAAG
CACTGGCGATCCCGGTTATTGGCATTGGCGCAGGCAACGTCACTGACGGGC
AGATCCTCG
TGATGCACGACGCCTTTGGTATTACCGGCGGTCACATTCCTAAATTCGCTAA
AAATTTCC
TCGCCGAAACGGGCGACATCCGCGCGGCTGTGCGGCAGTATATGGCTGAA
GTGGAGTCCG
GCGTTTATCCGGGCGAAGAACACAGTTTCCATTAAGGAGTCACGTTGTGTT
AATTA
TCGAAACCCTGCCGCTGCTGCGTCAGCAAATTCGCCGCCTGCGTATGGAAG
GCAAGCGCG
TGGCGCTGGTGCCTACCATGGGTAACCTGCACGATGGCCATATGAAGCTGG
TCGACGAAG
CCAAAGCCCGCGCCGATGTGGTCGTCGTCAGTATTTTCGTTAACCCGATGC
AGTTCGACC
GCCCGGAAGATCTGGCTCGTTATCCACGGACCTTGCAGGAGGACTGCGAG
AAGCTAAACA
AACGTAAAGTGGATTTAGTTTTCGCCCCTTCGGTAAAAGAGATCTACCCGA
ACGGTACTG
AAACCCACACTTACGTTGACGTTCCTGGCCTTTCGACCATGCTGGAAGGTG
CCAGCCGTC
CGGGACATTTTCGCGGCGTTTCGACTATTGTCAGCAAGCTGTTCAACCTGG
TCCAGCCGG
ACATCGCCTGCTTCGGTGAAAAAGATTTTCAGCAACTGGCGCTGATCCGCA
AAATGGTTG
CCGATATGGGCTTCGATATTGAGATTGTCGGTGTGCCAATTATGCGCGCCAA
 
AGACGGTC
TGGCGCTAAGTTCCCGTAACGGTTATCTGACGGCGGAACAACGCAAAATTG
CGCCTGGTC
TGTACAAAGTTTTAAGTTCGATTGCTGACAAATTGCAGGCTGGGGAACGGG
ATCTCGATG
AAATTATTACCATTGCGGGGCAAGAACTGAATGAAAAAGGCTTCCGCGCCG
ATGATATTC
AGATTCGCGATGCCGACACATTGCTGGAAGTTTCTGAAACCAGCAAACGG
GCAGTAATTC
TGGTAGCCGCCTGGCTTGGCGATGCTCGCCTGATCGACAACAAAATGGTCG
AGCTGGCGT
AATACTTAACTGGCGCTACGGCTGATGGCGCCAGTTATTAATTTACCCCACG
TCATCTGT
CATAACACAGG
**SEQ ID No.4** 
ACATGCATGC ttgacagctagctcagtcctaggtataatgctagc
GTTGCGGGTGAGGAGGAACAATGAAAATTACCGTATTGGGATGCGGTGCCT
TAGGGCAAT
TATGGCTTACAGCACTTTGCAAACAGGGTCATGAAGTTCAGGGCTGGCTGC
GCGTACCGC
AACCTTATTGTAGCGTGAATCTGGTTGAGACAGATGGTTCGATATTTAACGA
ATCGCTGA
CCGCCAACGATCCCGATTTTCTCGCCACCAGCGATCTGCTCCTGGTGACGC
TGAAAGCAT
GGCAGGTTTCCGATGCCGTCAAAAGCCTCGCGTCCACACTGCCTGTAACTA
CGCCAATAC
TGTTAATTCACAACGGCATGGGCACCATCGAAGAGTTGCAAAACATTCAGC
AGCCATTAC
TGATGGGCACCACCACCCATGCAGCCCGCCGCGACGGCAATGTCATTATTC
ATGTGGCAA
 
ACGGTATCACGCATATTGGCCCGGCACGGCAACAGGACGGGGATTACAGTT
ATCTGGCGG
ATATTTTGCAAACCGTGTTGCCTGACGTTGCCTGGCATAACAATATTCGCGC
CGAGCTGT
GGCGCAAGCTGGCAGTCAACTGCGTGATTAATCCACTGACTGCCATCTGGA
ATTGCCCGA
ACGGTGAATTACGTCATCATCCGCAAGAAATTATGCAGATATGCGAAGAAG
TCGCGGCGG
TGATCGAACGCGAAGGGCATCATACTTCAGCAGAAGATTTGCGTGATTACG
TGATGCAGG
TGATTGATGCCACAGCGGAAAATATCTCGTCGATGTTGCAGGATATCCGCGC
GCTGCGCC
ACACTGAAATCGACTATATCAATGGTTTTCTCTTACGCCGCGCCCGCGCGCA
TGGGATTG
CCGTACCGGAAAACACCCGCCTGTTTGAAATGGTAAAAAGAAAGGAGAGT
GAATATGAGC
GCATCGGCACTGGTTTGCCTCGCCCCTGGTAGTGAAGAGACTAATTTTCGA
AAAAAGACGCTGAAAAGCGTCTTTTTTCGTTTTGGGATCTAAATCGAG
**SEQ ID No.5** 
CG GACTGGAAGA AGATCTGTTT GTCTCTGCGC
GTCCGAATAT TGAACTGCTG CCGGAAGGCC AGTTTAAATA CCACGTCGAT
TTTGAGCATC
TGCATATTGG CGAAGAAACC GGGATGATTT GCGTCTCCCG GCCGACGAAT
CCAACAGGCA
ATGTGATTAC TGACGAAGAG TTGCTGAAGC TTGACGCGCT
GGCGAATCAA CACGGCATTC
CGCTGGTGAT TGATAACGCT TATGGCGTCC CGTTCCCGGG TATCATCTTC
AGTGAAGCGC
GCCCGCTATG GAATCCGAAT ATCGTGCTGT GCATGAGTCT
CAATTCCGACGTCTAAGAAACCATT
 
ATTATCATGACATTAACCTATAAAAA
TAGGCGTATCACGAGGCCCTTTCGTC
TTCACCTCGAGTCCCTATCAGTGATA
GAGATTGACATCCCTATCAGTGATAG
AGATACTGAGCACATCAGCAGGACG
CACTGACC
GGGCCCAAGTTCACTTAAAAAGGAGATCAACAATGAAAGCAATTTTCGTAC
TGAAACATCTTAATCATGCTAAGGAGGTTTTCTA
ATGTACCGTA CGTTAATGAG CGCAAAACTT CACAGAGCGA
GAGTGACGGA AGCCAATTTG
AACTACGTCG GCAGCGTGAC AATTGATGAA GATTTGCTGG ATGCTGTCGG
AATGATGGCA
AATGAAAAAG TGCAAATTGT GAATAATAAT AACGGGGCCC
GGCTGGAAAC GTACATTATT
CCCGGTGAAA GGGGCAGCGG CGTCGTTTGT TTAAACGGAG
CTGCCGCCCG CCTTGTCCAG
GTTGGAGATG TCGTCATCAT CGTGTCTTAT GCGATGATGT CTGAAGAGGA
AGCAAAGACC
CATAAGCCGA AGGTTGCCGT TTTGAACGAG AGAAACGAAA
TCGAGGAAAT GCTGGGTCAG
GAGCCAGCCC GTACCATTCT GTAA
AAGC
CTAGCATAACCCCTTGGGGCCTCTAAACGGGTCTTGAGGGGTTTTTTG
CATC ACCAATATGAACGGCA
TTAT CAGCCTGGCA CCTGGCGGTA TTGGTCCGGC GATGATGTGT
GAAATGATTA
AGCGTAACGA TCTGCTGCGC CTGTCTGAAA CAGTCATCAA ACCGTTTTAC
TACCAGCGTG
TTCAGGAAAC TATCGCCATC ATTCGCCGCT ATTTACCGGA AAATCGCTGC
CTGATTCATA
 
AACCGGAAGG AGCCATTTTC CTCTGGCTAT GGTTTAAGGA TTTGCCCATT
ACGACCAAGC
AGCTCTATCA GC
**SEQ ID No.6** 
GGCAGAAAAT CAGCCAGTTC CGCGCCCGCC ATCCCGCAAT
TGGCGCGGGC AAACAAACGA
CACTTTTGCT GAAGCAGGGC TACGGCTTTG TTCGTGAGCA TGGCGACGAT
AAAGTGCTGG
TCGTCTGGGC AGGGCAACAG TAACTTTTCC GGCTTCCCGT TCGTCAGTAC
CTCGGGAAGC
CGCCAACCAG GATAAAATGT CAGCCCTAAT CAGCGTTGCA
GGATAAAGCA CCGCTCACTC
TTCAACAGAC CGATTTGCAC CCCAGCAAAT GTAGCGTTAT TGTTACCTTC
CTTGCTACAG
AGTTCTTGA CAATTAATCATCCGGCTCGTATAATGTGTGGA
CAAGATT CAGGACGGGG AACTAACTAT G
AATGGCGCACAGTGGGTGGTACATGCGTTGCGGGCACAGGGTGTGAACAC
CGTTTTCGGTTATCCGGGTGGCGCAATTATGCCGGTTTACGATGCATTGTATG
ACGGCGGCGTGGAGCACTTGCTATGCCGACATGAGCAGGGTGCGGCAATG
GCGGCTATCGGTTATGCTCGTGCTACCGGCAAAACTGGCGTATGTATCGCCA
CGTCTGGTCCGGGCGCAACCAACCTGATAACCGGGCTTGCGGACGCACTG
TTAGATTCCATCCCTGTTGTTGCCATCACCGGTCAAGTGTCCGCACCGTTTA
TCGGCACTGACGCATTTCAGGAAGTGGATGTCCTGGGATTGTCGTTAGCCT
GTACCAAGCACAGCTTTCTGGTGCAGTCGCTGGAAGAGTTGCCGCGCATCA
TGGCTGAAGCATTCGACGTTGCCTGCTCAGGTCGTCCTGGTCCGGTTCTGG
TCGATATCCCAAAAGATATCCAGTTAGCCAGCGGTGACCTGGAACCGTGGT
TCACCACCGTTGAAAACGAAGTGACTTTCCCACATGCCGAAGTTGAGCAA
GCGCGCCAGATGCTGGCAAAAGCGCAAAAACCGATGCTGTACGTTGGCGG
TGGCGTGGGTATGGCGCAGGCAGTTCCGGCTTTGCGTGAATTTCTCGCTGC
CACAAAAATGCCTGCCACCTGTACGCTGAAAGGGCTGGGCGCAGTAGAAG
 
CAGATTATCCGTACTATCTGGGCATGCTGGGAATGCATGGCACCAAAGCGG
CGAACTTCGCGGTGCAGGAGTGCGACTTGCTGATCGCCGTGGGTGCACGT
TTTGATGACCGGGTGACCGGCAAACTGAACACCTTCGCACCACACGCCAG
TGTTATCCATATGGATATCGACCCGGCAGAAATGAACAAGCTGCGTCAGGC
ACATGTGGCATTACAAGGTGATTTAAATGCTCTGTTACCAGCATTACAGCAG
CCGTTAAATATCAATGACTGGCAGCTACACTGCGCGCAGCTGCGTGATGAA
CATGCCTGGCGTTACGACCATCCCGGTGACGCTATCTACGCGCCGTTGTTGT
TAAAACAACTGTCAGATCGTAAACCTGCGGATTGCGTCGTGACCACAGATG
TGGGGCAGCACCAGATGTGGGCTGCGCAGCACATCGCCCACACTCGCCCG
GAAAATTTCATCACCTCCAGCGGCTTAGGCACCATGGGTTTTGGTTTACCG
GCGGCGGTTGGCGCGCAAGTCGCGCGACCAAACGATACCGTCGTCTGTATC
TCCGGTGACGGCTCTTTCATGATGAATGTGCAAGAGCTGGGCACCGTAAAA
CGCAAGCAGTTACCGTTGAAAATCGTCTTACTCGATAACCAACGGTTAGGG
ATGGTTCGACAATGGCAGCAACTGTTTTTCCAGGAACGATATAGCGAAACC
ACCCTTACCGATAACCCCGATTTCCTCATGTTAGCCAGCGCCTTCGGCATCC
CTGGCCAACACATCACCCGTAAAGACCAGGTTGAAGCGGCACTCGACACC
ATGCTGAACAGTGATGGGCCATACCTGCTTCATGTCTCAATCGACGAACTT
GAGAACGTCTGGCCGCTGGTGCCGCCTGGTGCCAGTAATTCAGAAATGTTG
GAGAAATTATCATGATGCAACATCAGGTCAATGTATCGGCTCGCTTCAATCC
AGAAACCTTAGAACGTGTTTTACGCGTGGTGCGTCATCGTGGTTTCCACGT
CTGCTCAATGAATATGGCCGCCGCCAGCGATGCACAAAATATAAATATCGAA
TTGACCGTTGCCAGCCCACGGTCGGTCGACTTACTGTTTAGTCAGTTAAATA
AACTGGTGGACGTCGCACACGTTGCCATCTGCCAGAGCACAACCACATCA
CAACAAATCCGCGCCTGAGCG
CAAAAGGAATATACCAGAAAAGAGACGCTTTTAGAGCGTCTTTTTTCGTTT
T
GGAGCTACTC ACACTGCTTG CCGGAATGCT GCGCGAGAAG
TTGGGTTGGG
ATATCGAACC ACAGAATATT GCACTAACAA ACGGCAGCCA GAGCGCGTTT
TTCTACTTAT
 
TTAACCTGTT TGCCGGACGC CGTGCCGATG GTCGGGTCAA
AAAAGTGCTG TTCCCGCTTG
CACCGGAATA CATTGGCTAT GCTGACGCCG GACTGGAAGA AGATCTGTTT
GTCTCTGCGC
GTCCGAATAT TGAACTGCTG CCGGAAGGCC AGTTTAAATA CCACGTCGAT
TTTGAGCATC
TGCATATTGG C
**SEQ ID No.7** RBS-aspDH-ter L3S2P56 
GTTGCGT GTTCTGCTTC ATCGCGCTGA TGGGCGCAAG
CTCCTTCGAG CTGGCAGGTA CCTTCATCGT CAGCCTGATT ATCCTGATGT
TCTACGCTCG
CAAAATGCAC GAGCGCCAGA GCCACTCAAT GGATAACCAC
ACCGCGTCTA ACGCACATTA
ATTAAAAGTA TTTTCCGAGG CTCCTCCTTT CATTTTGTCC CATGTGTTGG
GAG
CA AAACATTAAC
ACCAACTGGC AAAATTTTGT CCTAAACTTG ATCTCGACGA AATGGCTGCA
CCTAAATCGT
GATGAAAATC ACATTTTTAT CGTAATTGCC CTTTAAAATT CGGGGCGCCG
ACCCCATGTG
GTCTCAAGCC CAAAGGAAGA GTGAGGCGAG TCAGTCGCGT
AATGCTTAGG CACAGGATTG
ATTTGTCGCA ATGATTGACA CGATTCCGCT TGACGCTGCG TAAGGTTTTT
GTAATTTTAC
AGGCAACCTT TTATTCAC
CATCTTAATCATGCTAAGGAGGTTTTCTAATGAATATTGCTGTGATTGGCTGC
GGTGCGATTGGCGCCA
GCGTGCTCGAACTGCTCAAGGGCCATGCCGCGGTGCAGGTGGGCTGGGTG
CTTGTGCCCG
AAGTGACGGACGCCGTGCGCGCCACCCTGGCCCGGCATGCGCCCCAGGCG
 
CGCGCACTGC
CTGCGCTGACGACTGAAGACCGGCCCGACCTTATCGTCGAATGCGCAGGC
CATACCGCCA
TCGAAGAGCATGTGCTGCCCGCCCTGCGGCGCGGCATTCCTGCCGTCGTGG
CCTCCATCG
GCGCACTCAGCGCCCCCGGCATGGCCGAGGCCGTTCAGGCCGCGGCCGAG
GCCGGAGGCA
CCCAGGTGCAATTGCTGTCGGGCGCCATCGGCGGCGTGGATGCGCTGGCCG
CAGCCCGCA
TCGGCGGCCTGGACGAAGTGGTCTACACCGGCCGCAAGCCGCCCCTGGCC
TGGACCGGCA
CGCCCGCAGAACAGCGCTGCGACCTCGCCAGCCTCAAGGAAGCCTTCTGC
ATCTTCGAAG
GCAGCGCACGCGAGGCCGCCCAGCTCTACCCCAAGAACGCCAACGTGGCC
GCCACCCTGT
CGCTGGCCGGCATGGGCCTGGACCGCACCACGGTGCGCCTGTACGCCGAC
CCGGCCGTGG
ACGAAAACGTGCACCATGTGGCCGCGCGCGGCGCCTTCGGTTCCATGGAAT
TGACCATGC
GCGGCAAGCCGCTGGAGGCCAACCCCAAGACCTCGGCCCTCACCGTCTAC
AGCGTGGTGC
GCGCCGTGCTCAACCAGGCCACGGCCATCGCCATCTAAGCCGCACCTTTTC
GAAAAAAGACGCTGAAAAGCGTCTTTTTTCGTTTTGGTCC
AGCTGTG CGAAGAAATT AGCAAAATGA
ACGAGAACCT GCCGTTGTAC GCGTTCGCTA ATACGTATTC CACTCTCGAT
GTAAGCCTGA
ATGACCTGCG TTTACAGATT AGCTTCTTTG AATATGCGCT GGGTGCTGCT
GAAGATATTG
CTAATAAGAT CAAGCAGACC ACTGACGA
**SEQ ID No.8** 
GTTGCGT GTTCTGCTTC ATCGCGCTGA TGGGCGCAAG
CTCCTTCGAG CTGGCAGGTA CCTTCATCGT CAGCCTGATT ATCCTGATGT
TCTACGCTCG
CAAAATGCAC GAGCGCCAGA GCCACTCAAT GGATAACCAC
ACCGCGTCTA ACGCACATTA
ATTAAAAGTA TTTTCCGAGG CTCCTCCTTT CATTTTGTCC CATGTGTTGG
GAG
CA AAACATTAAC
ACCAACTGGC AAAATTTTGT CCTAAACTTG ATCTCGACGA AATGGCTGCA
CCTAAATCGT
GATGAAAATC ACATTTTTAT CGTAATTGCC CTTTAAAATT CGGGGCGCCG
ACCCCATGTG
GTCTCAAGCC CAAAGGAAGA GTGAGGCGAG TCAGTCGCGT
AATGCTTAGG CACAGGATTG
ATTTGTCGCA ATGATTGACA CGATTCCGCT TGACGCTGCG TAAGGTTTTT
GTAATTTTAC
AGGCAACCTT TTATTCAC
TATACCTTCC AGAGCAATCT CACGTCTTGC AAAAACAGCC TGCGTTTTCA
TCAGTAATAG TTGGAATTTT GTAAATCTCC CGTTACCCTG ATAGCGGACT
TCCCTTCTGT
AACCATAATG GAACCTCGTC atgTTTGAGA ACATTACCGC CGCTCCTGCC
GACCCGATTC
TGGGCCTGGC CGATCTGTTT CGTGCCGATG AACGTCCCGG CAAAATTAAC
CTCGGGATTG
GTGTCTATAA AGATGAGACG GGCAAAACCC CGGTACTGAC
CAGCGTGAAA AAGGCTGAAC
AGTATCTGCT CGAAAATGAA ACCACCAAAA ATTACCTCGG CATTGACGGC
ATCCCTGAAT
TTGGTCGCTG CACTCAGGAA CTGCTGTTTG GTAAAGGTAG CGCCCTGATC
AATGACAAAC
 
GTGCTCGCAC GGCACAGACT CCGGGGGGCA CTGGCGCACT
ACGCGTGGCT GCCGATTTCC
TGGCAAAAAA TACCAGCGTT AAGCGTGTGT GGGTGAGCAA
CCCAAGCTGG CCGAACCATA
AGAGCGTCTT TAACTCTGCA GGTCTGGAAG TTCGTGAATA CGCTTATTAT
GATGCGGAAA
ATCACACTCT TGACTTCGAT GCACTGATTA ACAGCCTGAA TGAAGCTCAG
GCTGGCGACG
TAGTGCTGTT CCATGGCTGC TGCCATAACC CAACCGGTAT CGACCCTACG
CTGGAACAAT
GGCAAACACT GGCACAACTC TCCGTTGAGA AAGGCTGGTT
ACCGCTGTTT GACTTCGCTT
ACCAGGGTTT TGCCCGTGGT CTGGAAGAAG ATGCTGAAGG
ACTGCGCGCT TTCGCGGCTA
TGCATAAAGA GCTGATTGTT GCCAGTTCCT ACTCTAAAAA CTTTGGCCTG
TACAACGAGC
GTGTTGGCGC TTGTACTCTG GTTGCTGCCG ACAGTGAAAC CGTTGATCGC
GCATTCAGCC
AAATGAAAGC GGCGATTCGC GCTAACTACT CTAACCCACC
AGCACACGGC GCTTCTGTTG
TTGCCACCAT CCTGAGCAAC GATGCGTTAC GTGCGATTTG
GGAACAAGAG CTGACTGATA
TGCGCCAGCG TATTCAGCGT ATGCGTCAGT TGTTCGTCAA TACGCTGCAG
GAAAAAGGCG
CAAACCGCGA CTTCAGCTTT ATCATCAAAC AGAACGGCAT GTTCTCCTTC
AGTGGCCTGA
CAAAAGAACA AGTGCTGCGT CTGCGCGAAG AGTTTGGCGT
ATATGCGGTT GCTTCTGGTC
GCGTAAATGT GGCCGGGATG ACACCAGATA ACATGGCTCC GCTGTGCGAA
GCGATTGTGG
 
CAGTGCTGta aGCATTAAAA ACAATGAAGC CCGCTGAAAA GCGGGCTGAG
ACTGATGACA
AACGCAACAT TGCCTGATGC GCTACGCTTA TCAGGCCTAC GCGTCCCCTG
CAATATTTTG
AATTTGCACG ATTTTGTAGG CCGGATAAGG CGCTCGTGCC GCATCCGGCA
TAAACAAAGC
GCACTTTGCC AGAAATCCAG G
AGCTGTG CGAAGAAATT AGCAAAATGA
ACGAGAACCT GCCGTTGTAC GCGTTCGCTA ATACGTATTC CACTCTCGAT
GTAAGCCTGA
ATGACCTGCG TTTACAGATT AGCTTCTTTG AATATGCGCT GGGTGCTGCT
GAAGATATTG
CTAATAAGAT CAAGCAGACC ACTGACGA
**SEQ ID No.9** 
GTTGCGT GTTCTGCTTC ATCGCGCTGA TGGGCGCAAG
CTCCTTCGAG CTGGCAGGTA CCTTCATCGT CAGCCTGATT ATCCTGATGT
TCTACGCTCG
CAAAATGCAC GAGCGCCAGA GCCACTCAAT GGATAACCAC
ACCGCGTCTA ACGCACATTA
ATTAAAAGTA TTTTCCGAGG CTCCTCCTTT CATTTTGTCC CATGTGTTGG
GAG
CA AAACATTAAC
ACCAACTGGC AAAATTTTGT CCTAAACTTG ATCTCGACGA AATGGCTGCA
CCTAAATCGT
GATGAAAATC ACATTTTTAT CGTAATTGCC CTTTAAAATT CGGGGCGCCG
ACCCCATGTG
GTCTCAAGCC CAAAGGAAGA GTGAGGCGAG TCAGTCGCGT
AATGCTTAGG CACAGGATTG
ATTTGTCGCA ATGATTGACA CGATTCCGCT TGACGCTGCG TAAGGTTTTT
GTAATTTTAC
 
AGGCAACCTT TTATTCAC
GCA
GCTTGAAAAA GAAGGTTCAC atgTCAAACA ACATTCGTAT CGAAGAAGAT
CTGTTGGGTA
CCAGGGAAGT TCCAGCTGAT GCCTACTATG GTGTTCACAC TCTGAGAGCG
ATTGAAAACT
TCTATATCAG CAACAACAAA ATCAGTGATA TTCCTGAATT TGTTCGCGGT
ATGGTAATGG
TTAAAAAAGC CGCAGCTATG GCAAACAAAG AGCTGCAAAC
CATTCCTAAA AGTGTAGCGA
ATGCCATCAT TGCCGCATGT GATGAAGTCC TGAACAACGG AAAATGCATG
GATCAGTTCC
CGGTAGACGT CTACCAGGGC GGCGCAGGTA CTTCCGTAAA
CATGAACACC AACGAAGTGC
TGGCCAATAT CGGTCTGGAA CTGATGGGTC ACCAAAAAGG TGAATATCAG
TACCTGAACC
CGAACGACCA TGTTAACAAA TGTCAGTCCA CTAACGACGC
CTACCCGACC GGTTTCCGTA
TCGCAGTTTA CTCTTCCCTG ATTAAGCTGG TAGATGCGAT TAACCAACTG
CGTGAAGGCT
TTGAACGTAA AGCTGTCGAA TTCCAGGACA TCCTGAAAAT GGGTCGTACC
CAGCTGCAGG
ACGCAGTACC GATGACCCTC GGTCAGGAAT TCCGCGCTTT CAGCATCCTG
CTGAAAGAAG
AAGTGAAAAA CATCCAACGT ACCGCTGAAC TGCTGCTGGA
AGTTAACCTT GGTGCAACAG
CAATCGGTAC TGGTCTGAAC ACGCCGAAAG AGTACTCTCC
GCTGGCAGTG AAAAAACTGG
CTGAAGTTAC TGGCTTCCCA TGCGTACCGG CTGAAGACCT
GATCGAAGCG ACCTCTGACT
 
GCGGCGCTTA TGTTATGGTT CACGGCGCGC TGAAACGCCT
GGCTGTGAAG ATGTCCAAAA
TCTGTAACGA CCTGCGCTTG CTCTCTTCAG GCCCACGTGC CGGCCTGAAC
GAGATCAACC
TGCCGGAACT GCAGGCGGGC TCTTCCATCA TGCCAGCTAA
AGTAAACCCG GTTGTTCCGG
AAGTGGTTAA CCAGGTATGC TTCAAAGTCA TCGGTAACGA CACCACTGTT
ACCATGGCAG
CAGAAGCAGG TCAGCTGCAG TTGAACGTTA TGGAGCCGGT
CATTGGCCAG GCCATGTTCG
AATCCGTTCA CATTCTGACC AACGCTTGCT ACAACCTGCT GGAAAAATGC
ATTAACGGCA
TCACTGCTAA CAAAGAAGTG TGCGAAGGTT ACGTTTACAA CTCTATCGGT
ATCGTTACTT
ACCTGAACCC GTTCATCGGT CACCACAACG GTGACATCGT
GGGTAAAATC TGTGCCGAAA
CCGGTAAGAG TGTACGTGAA GTCGTTCTGG AACGCGGTCT
GTTGACTGAA GCGGAACTTG
ACGATATTTT CTCCGTACAG AATCTGATGC ACCCGGCTTA CAAAGCAAAA
CGCTATACTG
ATGAAAGCGA ACAGtaaTCG TACAGGGTAG TACAAATAAA AAAGGCACGT
CAGATGACGT
GCCTTTTTTC TTGTGAGCAG AGCTGTG CGAAGAAATT AGCAAAATGA
ACGAGAACCT GCCGTTGTAC GCGTTCGCTA ATACGTATTC CACTCTCGAT
GTAAGCCTGA
ATGACCTGCG TTTACAGATT AGCTTCTTTG AATATGCGCT GGGTGCTGCT
GAAGATATTG
CTAATAAGAT CAAGCAGACC ACTGACGA
**SEQ ID No.56**

ATGAATATTGCTGTGATTGGCTGCGGTGCGATTGGCGCCA

GCGTGCTCGAACTGCTCAAGGGCCATGCCGCGGTGCAGGTGGGCTGGGTG
CTTGTGCCCG
AAGTGACGGACGCCGTGCGCGCCACCCTGGCCCGGCATGCGCCCCAGGCG
CGCGCACTGC
CTGCGCTGACGACTGAAGACCGGCCCGACCTTATCGTCGAATGCGCAGGC
CATACCGCCA
TCGAAGAGCATGTGCTGCCCGCCCTGCGGCGCGGCATTCCTGCCGTCGTGG
CCTCCATCG
GCGCACTCAGCGCCCCCGGCATGGCCGAGGCCGTTCAGGCCGCGGCCGAG
GCCGGAGGCA
CCCAGGTGCAATTGCTGTCGGGCGCCATCGGCGGCGTGGATGCGCTGGCCG
CAGCCCGCA
TCGGCGGCCTGGACGAAGTGGTCTACACCGGCCGCAAGCCGCCCCTGGCC
TGGACCGGCA
CGCCCGCAGAACAGCGCTGCGACCTCGCCAGCCTCAAGGAAGCCTTCTGC
ATCTTCGAAG
GCAGCGCACGCGAGGCCGCCCAGCTCTACCCCAAGAACGCCAACGTGGCC
GCCACCCTGT
CGCTGGCCGGCATGGGCCTGGACCGCACCACGGTGCGCCTGTACGCCGAC
CCGGCCGTGG
ACGAAAACGTGCACCATGTGGCCGCGCGCGGCGCCTTCGGTTCCATGGAAT
TGACCATGC
GCGGCAAGCCGCTGGAGGCCAACCCCAAGACCTCGGCCCTCACCGTCTAC
AGCGTGGTGC
GCGCCGTGCTCAACCAGGCCACGGCCATCGCCATCTAA

### Embodiment 3 Fermentation experiment of VB5 engineering bacteria

The test strains engineering bacteria *E. coli* MG1655 *avtA:panDBl-ilvG*+*M-aspDH*/pACYC184-*panBCE, E. coli* MG1655 *avtA:panDBl-ilvG*+*M-aspC*/pACYC184*-panBCE* and E. coli MG1655 *avtA: panDBl-ilvG*+*M-aspA*/pACYC184*-panBCE*, were streak-inoculated on solid LB medium plates containing 34 mg/L chloramphenicol, and incubated at 37°C for 12 hours. The bacterial moss on the plate was picked and inoculated into the slant of LB medium, and incubated at 37°C for 10-12 h. The bacterial moss on the plate was picked and inoculated into liquid LB medium, and cultured at 37°C and 220 rpm with shaking for 12 h to obtain the seed liquid. The seed liquid was inoculated into the fermentation medium in 3% inoculum, and cultured at 37°C, 220rpm with shaking.

Fermentation medium: MOPS 80 g/L, glucose 20.0 g/L, ammonium sulfate 10.0 g/L, potassium dihydrogen phosphate 2.0 g/L, magnesium sulfate heptahydrate 2.0 g/L, yeast 5.0 g/L, trace element mixed solution 5 mL/L, and the remaining amount was water. Trace element mixed solution: FeSO₄·7H₂O10g/L, CaCl₂1.35g/L, ZnSO₄·7H₂O2.25g/L, MnSO₄·4H₂O0.5g/L, CuSO₄·5H₂O1g/L, (NH₄)₆Mo₇O₂₄·4H₂O0.106g/L, Na₂B₄O₇·10H₂O0.23g/L, CoCl₂·6H₂O0.48g/L, 35% HCl10mL/L, and the remaining amount was water.

During the incubation process, samples were taken every 4 h. The pH of the reaction system was adjusted with ammonia to maintain it at 6.8-7.0. Glucose content was detected using a biosensor analyzer SBA-40D, and when the glucose content of the system was less than 5 g/L, glucose was added to make the glucose concentration of the system reach 20 g/L. Samples were taken after 24 h of incubation, centrifuged at 12,000g for 2 min, and the supernatant was taken to detect the VB5 content. The supernatant was taken and tested for VB5 content (as follows).

**Table 1**

| engineering bacteria | Vitamins B5 ( g/L ) |
|---|---|
| *E. coli* MG1655 *avtA:panDBl-ilvG*⁺*M-aspDH*/pACYC184*-panBCE* | 4.86±0.13 |
| *E. coli* MG1655 | 3.55±0.25 |
| *avtA:panDBl-ilvG*⁺*M-aspC*/pACYC184*-panBCE* | |
| *E. coli* MG1655 *avtA:panDBl-ilvG*⁺*M*-*aspA*/pACYC184*-panBCE* | 3.97±0.15 |

The present application enhances three different pathways for aspartic acid production in E. coli for VB5 production by fermentation, found that engineering bacteria overexpressing the *aspDH* gene is more favorable to increase the fermentation yield of VB5 than overexpressing *aspC* and *aspA.*

The foregoing is only a preferred embodiment of the present invention, and it should be noted that the skilled in the art can make several improvements and embellishments without departing from the principle of the present invention, and these improvements and embellishments should also be regarded as the protection scope of the present invention.

## Claims

1. An application of enhanced expression of an aspartate dehydrogenase gene *aspDH* in the production of vitamin B5;
preferably, the aspartate dehydrogenase gene *aspDH* is derived from *Delftia sp. Csl-4.*

2. The application according to claim 1, wherein the aspartate dehydrogenase gene *aspDH* has:
(I) a nucleotide sequence shown as SEQ ID No. 56; or
(II) a nucleotide sequence obtained by substituting, deleting or adding one or more bases to the nucleotide sequence shown as (I) and having the same or similar functions as the nucleotide sequence shown as (I); or
(III) a nucleotide sequence having at least 80% homology to the nucleotide sequence shown as (I) or (II).

3. The application according to claim 1 or 2, further comprising:
(1) inserting a strong promoter and/or a strong RBS into a *cadA* gene, wherein the strong promoter is PgapA and the strong RBS is BCD2;
preferably, the BCD2 has:
(A) a nucleotide sequence shown as SEQ ID No. 2; or
(B) a nucleotide sequence obtained by substituting, deleting or adding one or more bases to the nucleotide sequence shown as (A), and having the same or similar function as the nucleotide sequence shown as (A); or
(C) a nucleotide sequence having at least 80% homology to the nucleotide sequence shown as (A) or (B);
and/or
(2) expressing an *ilvGM* gene derived from E. coli BL21; and/or
(3) expressing a L-aspartate α-decarboxylase gene *panD* derived from Bacillus *licheniformis;* and/or
preferably, the L-aspartate α-decarboxylase gene *panD* derived from *Bacillus licheniformis* has:
(A) a nucleotide sequence shown as SEQ ID No. 1; or
(B) a nucleotide sequence obtained by substituting, deleting or adding one or more bases to the nucleotide sequence shown as (A), and having the same or similar function as the nucleotide sequence shown as (A); or
(C) a nucleotide sequence having at least 80% homology to the nucleotide sequence shown as (A) or (B); and/or
(4) increasing copy number *of a panB* gene, *a panC* gene and/or *a panE* gene.

4. An expression vector, comprising an aspartate dehydrogenase gene *aspDH,*
preferably, the aspartate dehydrogenase gene *aspDH* is derived from *Delftia sp. Csl-4 ;*
preferably, the aspartate dehydrogenase gene *aspDH* has:
(I) a nucleotide sequence shown as SEQ ID No. 56; or
(II) a nucleotide sequence obtained by substituting, deleting or adding one or more bases to the nucleotide sequence shown as (I), and having the same or similar function as the nucleotide sequence shown as (I); or
(III) a nucleotide sequence having at least 80% homology to the nucleotide sequence shown as (I) or (II).

5. The expression vector according to claim 4, further comprising:
(I) a strong promoter and/or a strong RBS;
wherein the strong promoter is PgapA and the strong RBS is BCD2;
preferably, the BCD2 has:
(A) a nucleotide sequence shown as SEQ ID No. 2; or
(B) a nucleotide sequence obtained by substituting, deleting or adding one or more bases to the nucleotide sequence shown as (A), and having the same or similar function as the nucleotide sequence shown as (A); or
(C) a nucleotide sequence having at least 80% homology to the nucleotide sequence shown as (A) or (B);
and/or
(II) an *ilvGM* gene derived from E. coli BL21; and/or
(III) a L-aspartate α-decarboxylase gene *panD* derived from *Bacillus licheniformis;* and/or
preferably, the L-aspartate α-decarboxylase gene *panD* derived from *Bacillus licheniformis* has:
(A) a nucleotide sequence shown as SEQ ID No. 1; or
(B) a nucleotide sequence obtained by substituting, deleting or adding one or more bases to the nucleotide sequence shown as (A), and having the same or similar function as the nucleotide sequence shown as (A); or
(C) a nucleotide sequence having at least 80% homology to the nucleotide sequence shown as (A) or (B); and/or
(IV) a *panB* gene, a *panC* gene and/or a *panE* gene with increased copy number.

6. A host, wherein the host expresses an aspartate dehydrogenase gene *aspDH,*
preferably, the aspartate dehydrogenase gene *aspDH* is derived from *Delftia sp. Csl-4;*
preferably, the aspartate dehydrogenase gene *aspDH* has:
(I) a nucleotide sequence shown as SEQ ID No. 56; or
(II) a nucleotide sequence obtained by substituting, deleting or adding one or more bases to the nucleotide sequence shown as (I), and having the same or similar function as the nucleotide sequence shown as (I); or
(III) a nucleotide sequence having at least 80% homology to the nucleotide sequence shown as (I) or (II).

7. The host according to claim 6, further comprising:
(I) a strong promoter and/or a strong RBS;
wherein the strong promoter is PgapA and the strong RBS is BCD2; preferably, the BCD2 has:
(A) a nucleotide sequence shown as SEQ ID No. 2; or
(B) a nucleotide sequence obtained by substituting, deleting or adding one or more bases to the nucleotide sequence shown as (A), and having the same or similar function as the nucleotide sequence shown as (A); or
(C) a nucleotide sequence having at least 80% homology to the nucleotide sequence shown as (A) or (B);
and/or
(II) an *ilvGM* gene derived from E. coli BL21; and/or
(III) a L-aspartate α-decarboxylase gene *panD* derived from *Bacillus licheniformis;* and/or
preferably, the L-aspartate α-decarboxylase gene *panD* derived from *Bacillus licheniformis* has:
(A) a nucleotide sequence shown as SEQ ID No. 1; or
(B) a nucleotide sequence shown as (A) obtained by substitution, deletion or addition of one or more bases, and having the same or similar function as the nucleotide sequence shown as (A); or
(C) a nucleotide sequence having at least 80% homology to the nucleotide sequence shown in (A) or (B); and/or
(IV) a panB gene, a panC gene and/or a panE gene with increased copy number.

8. The host according to claim 6 or 7, wherein the expression vector according to claim 4 or 5 is transfected or transformed;
preferably the host is derived from E. coli,
more preferably the host is derived fromE. coli K12, and
more preferably the host is derived from E. coli K12 MG1655 strain.

9. The application of the expression vector according to claim 4 or 5, the host of any one of claims 6 to 8, in the production of vitamin B5.

10. A method for the production of vitamin B5, wherein the host according to any one of claims 6 to 8 is used as a fermentation strain, fermented, the fermentation broth is collected, and the supernatant is centrifuged to obtain vitamin B5.
